# EUROPEAN PATENT APPLICATION

(11) **EP 2 308 472 A1**
(43) Date of publication of application: **13.04.2011**
(21) Application number: 09012654.1
(22) Date of filing: 06.10.2009
(51) Int. Cl.: A61K 9/14, A61K 9/16, A61K 9/20, A61K 31/5377

(54) **Pharmaceutical compositions comprising rivaroxaban**

(71) Applicant: ratiopharm GmbH, 89079 Ulm (DE)
(72) Inventor: Stefan, Ralph, 88370 Ebenweiler (DE); Paetz, Jana, 82272 Moorenweis (DE)
(74) Representative: Aechter, Bernd

(57) **Abstract**

The invention relates to pharmaceutical compositions comprising rivaroxaban, suitable for immediate release and processes of preparing such compositions, preferably by a melt-granulation process.

## Description

The invention relates to pharmaceutical compositions comprising rivaroxaban, suitable for immediate release, and processes of preparing such compositions, preferably by a melt-granulation process.

5-Chloro-*N*-({(*5S*)-2-oxo-3-[4-(3-oxo-4-morpholinyl)-phenyl]-1,3-oxazolidin-5-yl}-methyl)-2-thiophencarboxamid is a low-molecular, orally administrable inhibitor of the blood coagulation factor Xa, investigated for the prophylaxis and/or treatment of various thromboembolic diseases (see WO 01/47919) and known under the INN rivaroxaban or under the trade name Xarelto^{®}. The 5-Chloro-*N*-({(*5S*)-2-oxo-3-[4-(3-oxo-4-morpholinyl)-phenyl]-1,3-oxazolidin-5-yl}-methyl)-2-thiophencarboxamid (= rivaroxaban) has the following chemical structure (I):

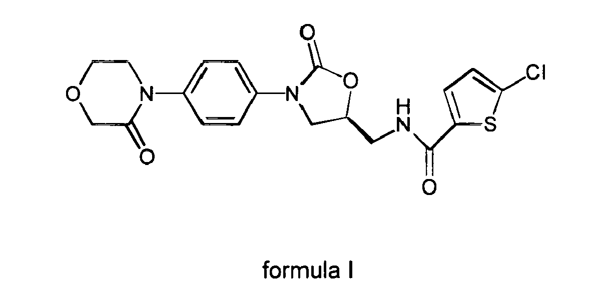

In this regard it is noted that the compound according to formula I refers to 5-Chloro-N-({(*5S*)-2-oxo-3-[4-(3-oxo-4-morpholinyl)-phenyl]-1,3-oxazolidin-5-yl}-methyl)-2-thiophencarboxamid (= rivaroxaban) or its solvates or hydrates as well as pharmaceutical acceptable salts thereof, preferably obtained according to the procedures as outlined in WO 01/47919. This form has been described in WO2007/039132 as crystalline form I.

In the art, several formulations of rivaroxaban are known. For example, formulations having modified release properties are described in WO 2006/072367.

Rivaroxaban has only limited solubility in water, causing problems regarding dissolution of the API from the pharmaceutical composition and the oral bioavailability.

In order to improve the bioavailability of rivaroxaban, several concepts have been put forward. WO 2005/060940 teaches the use of the wet granulation technique in combination with the use of hydrophilic matrix formers in order to hydrophilize the rivaroxaban and to improve bioavailability.

WO 2007/039122 or WO 2009/049820 discloses immediate release forms comprising the use of an amorphous or semi-stable crystalline modification of rivaroxaban as API. The use of these modifications increases the solubility and the oral bioavailability compared to the formulations described in WO2005/060940, using the rivaroxaban in crystalline modification I.

Employing the above hydrophilization by wet granulation approach, using the stable crystalline modification, rivaroxaban, does not provide sufficient bioavailability compared to using the amorphous state according to the teaching in WO 2007/039122. The use of rivaroxaban in the amorphous state is hampered by stability issues, due to the tendency of the amorphous form to switch to a semi-crystalline state. The wet granulation technique furthermore is energy- and time-consuming and cost-intensive.

It is therefore an object of the invention to provide a process for the manufacture of a pharmaceutical composition comprising rivaroxaban or a pharmaceutically acceptable salt thereof which does not encounter the above mentioned problems. In particular, a pharmaceutical composition should be provided, having improved properties like solubility, dissolution profile, stability, flowability and bioavailability. Especially, it was an object of the present invention to provide an immediate release pharmaceutical rivaroxaban composition having a superior dissolution profile even after prolonged time of storage. Preferably, complete drug release should be achieved, even after storage.

Furthermore, it has been found that the content uniformity of the pharmaceutical compositions as disclosed in WO 2005/060940 is still optimizable. Particularly, in the case of rivaroxaban, a superior content uniformity is desirable, since the interindividual variability in pharmacokinetics is significant and ranges from 30 % to 40 % (see Product Monograph Xarelto^{®}, 2008). Therefore, it was a further object of the present invention to provide pharmaceutical compositions comprising rivaroxaban suitable for having a superior dissolution profile and a superior high content uniformity.

Moreover, it has been found that the process as described in WO 2005/060940 is still optimizable with regard to operational health and safety, in particular with regard to the production of respirable dust. Hence, it was an object of the present invention to provide a process for preparing a rivaroxaban formulation, wherein the production of respirable dust is reduced or preferably completely avoided.

It has now been found that the above problems can be overcome by providing pharmaceutical formulations comprising rivaroxaban, a hydrophilic matrix former and a disintegrant, obtained by a melt-extrusion process.

The problem can further be overcome by a melt-granulation or melt-extrusion process for the manufacture of a pharmaceutical formulation of rivaroxaban or its solvates and hydrates.

Hence, a subject of the present invention is a process for producing a pharmaceutical composition, comprising the steps of
(i) mixing
   a) rivaroxaban,
   b) a matrix former, preferably a hydrophilic matrix former, and
   c) a disintegrant
(ii) melting the mixture, optionally cooling off, and
(iii) granulating the melted mixture.

A further subject of the present invention is a pharmaceutical composition obtainable by the process of the present invention. In addition, a further subject of the present invention are oral dosage forms, preferably tablets or capsules, containing the pharmaceutical composition of the present invention.

Moreover, another subject of the present invention is a process for producing tablets, comprising the steps of
(i) mixing
   a) rivaroxaban,
   b) a matrix former, preferably a hydrophilic matrix former,
      c1) a disintegrant, and
      d1) optionally wicking agent,
(ii) melting the mixture,
(iii) granulating the melted mixture,
(iv) mixing the granulate resulting from step (iii) with
   c2) disintegrant,
   d2) optionally wicking agent, and
   e) optionally, further excipients; and
(v) compressing the mixture resulting from step (iv) into tablets.

As well, tablets obtainable by said process are subjects of the present invention.

Finally, a subject of the present invention is the use of a combination of crospovidone and a wicking agent for producing an immediate release solid oral dosage form containing rivaroxaban.

The above illustrated subjects of the present invention are alternative solutions to the above outlined problems.

In the following, explanations regarding the pharmaceutical composition of the present invention are given. However, these explanations also apply to the oral dosage form of the present invention, to the use of the present invention and to the processes for producing the pharmaceutical composition or for producing the oral dosage form of the present invention.

In the pharmaceutical composition of the present invention, rivaroxaban as the active ingredient (component (a)) preferably is present in crystalline form, wherein the crystalline modification I as described in WO 2005/060940 is particularly preferred. Preferably, the active ingredient is present in the form of the free base.

In a preferred embodiment rivaroxaban as the active ingredient (a) is employed in a micronized form. That means, the active ingredient (a) of the pharmaceutical composition of the present invention has a volume mean particle size (D50) of 0.1 to 10 µm, more preferably of 0.5 to 5 µm, still more preferably of 1.0 to 4 µm.

Furthermore, in a preferred embodiment the D(90) value of the volume mean particle size distribution is from 1 to 15 µm, preferably from 2 to 10 µm, more preferably from 3 to 8 µm.

Furthermore, in a preferred embodiment the D(10) value of the volume mean particle size distribution is from 0.01 to 5 µm, preferably from 0.1 to 2.0 µm, more preferably from 0.5 to 1.0 µm.

Within this application, the volume mean particle size (D50) is determined by the light scattering method, using a Mastersizer 2000 apparatus made by Malvern Instruments (wet measurement, 2000 rpm, ultrasonic waves for 60 sec., data interpretation via Fraunhofer Method, Dispersant: 0.02% SDS solution, Obscuration:10 - 20 %, Stirrer speed: 2000 rpm, Stirring duration: 15min prior to first measurement cycle, Sonication: no, Background time: 10 sec, Measurement time: 10 sec, Measurement cycles: 3

The volume mean particle size (D50), which is also denoted D50 value of the integral volume distribution, is defined in the context of this invention as the particle diameter, at which 50 percent by volume of the particles have a smaller diameter than the diameter which corresponds to the D50 value. Likewise, 50 percent by volume of the particles have a larger diameter than the D50 value. Analogous, the D90 value of the integral volume distribution is defined as the particle diameter, at which 90 percent by volume of the particles have a smaller diameter than the diameter which corresponds to the D90 value. Correspondingly, the D10 value of the integral volume distribution is defined as the particle diameter, at which 10 percent by volume of the particles have a smaller diameter than the diameter which corresponds to the D10 value.

The pharmaceutical composition further comprises one or more matrix formers (b), preferably hydrophilic matrix formers (b). Generally, the term "matrix former" means any organic excipient, which is capable of forming a matrix in a melt extrusion process. Generally, the term "hydrophilic matrix former" means any organic excipient, which possesses hydrophilic groups and is capable of forming a matrix in a melt extrusion process. Preferably, the matrix former, preferably the hydrophilic matrix former, improves the solubility and dissolution of the active pharmaceutical ingredient. Preferably, the hydrophilic matrix former is capable of reducing the dissolution time of a pharmaceutical composition by 5 %, more preferably by 20 %, according to USP 31-NF26 release method, using apparatus 2 (paddle), compared to the same pharmaceutical composition comprising calcium hydrogen phosphate instead of the hydrophilic matrix former.

The matrix formers are selected, for example, from the group of known inorganic or organic excipients. Such excipients preferably include polymers, low molecular weight oligomers and natural products.

Preferably, the hydrophilic matrix former is a water-soluble compound, having a water solubility of more than 10 mg/l, more preferably of more than 20 mg/l, still more preferably of more than 50 mg/l at a temperature of 25 °C. The solubility of the hydrophilic matrix former might be e.g. up to 1,000 mg/l or up to 300 mg/ml at a temperature of 25 °C. The water-solubility is determined according to the column elution method of the Dangerous Substances Directive (67/548/EEC), Annex V, Chapter A6.

In a preferred embodiment the matrix former is a hydrophilic polymer, preferably having the above mentioned water-solubility. Generally, the term "hydrophilic polymer" encompasses polymers comprising polar groups. Examples for polar groups are hydroxy, amino, amido, carboxy, carbonyl, ether, ester and sulfonate. Amido groups are particularly preferred.

The hydrophilic polymer usually has a weight average molecular weight, ranging from 1,000 to 250,000 g/mol, preferably from 2,000 to 100,000 g/mol, particularly from 4,000 to 75,000 g/mol. Furthermore, a 2 % w/w solution of the hydrophilic polymer in pure water preferably has a viscosity of from 1 to 20 mPas, more preferably from 2 to 8 mPas at 25 °C. The viscosity is determined according to the European Pharmacopoeia (hereinafter referred to as Ph. Eur.), 6^{th} edition, Chapter 2.2.10.

Furthermore, the hydrophilic polymer used as hydrophilic matrix former preferably has a glass transition temperature (T_{g}) or a melting point of 25 °C to 200 °C, more preferably of 90 °C to 170 °C. The glass transition temperature, T_{g}, is the temperature at which the hydrophilic polymer becomes brittle on cooling and soft on heating. That means, above T_{g}, the hydrophilic polymers become soft and capable of plastic deformation without fracture. The glass transition temperature or the melting point are determined with a Mettler-Toledo^{®} DSC 1, wherein a heating rate of 10 °C per minute and a cooling rate of 15 °C per minute is applied. The determination method essentially is based on Ph.Eur. 6.1, section 2.2.34. For the determination of T_{g} the polymer is heated twice (i.e. heated, cooled, heated).

More preferably, derivatives of cellulose (e.g. hydroxyproply methyl cellulose (HPMC), preferably having a weight average molecular weight from 20,000 to 90,000 g/mol, and/or preferably a ratio of methyl groups from 10 to 35 %, and preferably a ratio of hydroxy groups from 1 to 35 %; hydroxypropyl cellulose (HPC), preferably having a weight average molecular weight of from 40,000 to 100,000 g/mol), polyvinylpyrrolidone, preferably having a weight average molecular weight of from 10,000 to 60,000 g/mol, copolymers of polyvinylpyrrolidones, preferably copolymers comprising vinylpyrrolidone and vinylacetate units (e.g. Povidon^{®} VA 64; BASF), preferably having a weight average molecular weight of 40,000 to 75,000 g/mol, polyoxyethylene alkyl ethers, co-blockpolymers of ethylene oxide and propylene oxide, preferably having a polyethylene content of 70 to 90 wt.% and/or preferably having a weight average molecular weight from 1,000 to 50,000 g/mol, in particular from 3,000 to 25,000 g/mol, polyvinyl alcohol, polyethylene glycol, preferably having a weight average molecular weight ranging from 1,000 to 50,000 g/mol are used as hydrophilic matrix formers. The weight average molecular weight is preferably determined by gel electrophoresis.

In particular, polyvinylpyrrolidone and copolymers of polyvinylpyrrolidone, in particular copolymers comprising vinylpyrrolidone and vinylacetate units having the structure 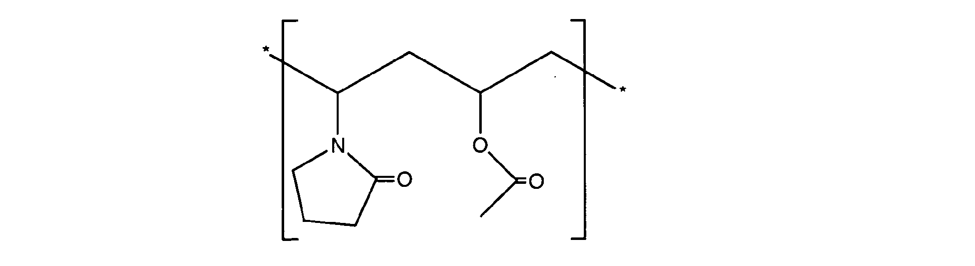 are used as hydrophilic matrix formers.

It is particularly preferred that the above mentioned kinds of hydrophilic polymers fulfill the functional requirements (molecular weight, viscosity, T_{g}, melting point, non-semipermeable properties) as illustrated above.

In the pharmaceutical composition of the present invention, at least one of the above mentioned hydrophilic matrix formers is present. Alternatively, a combination of two or more hydrophilic matrix formers can be employed.

Besides rivaroxaban (a) and matrix former (b), the pharmaceutical composition of the present invention comprises one or more disintegrants (c).

Generally, disintegrants (c) are compounds capable of promoting the break up of a solid composition into smaller pieces when the composition gets in contact with a liquid, preferably water.

Preferred disintegrants (c, c1 and/or c2) are sodium carboxymethyl starch, cross-linked polyvinylpyrrolidone (crospovidone), sodium carboxymethyl glycolate (e.g. Explotab^{®}), swelling polysaccharide, e.g. soya polysaccharide, carrageenan, agar, pectin, starch and derivates thereof, protein, e.g. formaldehyde - casein, sodium bicarbonate or mixtures thereof. Crospovidone is particularly preferred as disintegrant.

Besides rivaroxaban (a), matrix former (b) and disintegrants (c), the pharmaceutical composition of the present invention comprises in a preferred embodiment one or more wicking agents.

Generally, a wicking agent (d) is material with the ability to draw a biological fluid (preferably water) into a solid (preferably into the granulates resulting from step (iii) of the process of the present invention), preferably by physisorption. Physisorption is defined as a form of adsorption, in which the solvent molecules can loosely adhere to surfaces of the wicking agent, preferably via van der Waals interaction between the surface of the wicking agent and the adsorbed fluid molecule (preferably water). Usually, a wicking agent can do this with or without swelling. Preferably, the wicking agent is a swelling wicking agent. Usually, a non-swelling wicking agent that attracts water will ultimately have a volume that is essentially composed of the volume of the wicking agent and the volume of water attracted to it. Usually, a swelling wicking agent will have a volume that is essentially composed of the volume of the wicking agent, the volume of water attracted to it, and an additional volume created by steric and molecular forces.

Preferably, the wicking agent (d) comprised in the pharmaceutical composition of the present invention creates channels or pores in the granulates. This facilitates the channeling of water molecules through the granulates, particularly by physisorption. Hence, the function of the wicking agent is to carry water to surfaces inside the granulates, thereby creating channels or a network of increased surface area.

Examples of wicking agents that may be used include, but are not limited to, microcrystalline cellulose, silicified microcrystalline cellulose, colloidal silicone dioxide, kaolin, titanium dioxide, fumed silicone dioxide, alumina, niacinamide, m-pyrol, bentonite, magnesium aluminum silicate, polyester, polyethylene, or mixtures thereof. Preferably, the wicking agents used in the pharmaceutical composition of the present invention include cellulose and cellulose derivatives, such as silicified microcrystalline cellulose, colloidal silicone dioxide, and mixtures thereof. The silicified microcrystalline cellulose that is preferred is commercially available under the trade name Prosolv^{®}, having a silicone dioxide content from 1 to 3 wt.%, preferably of about 2 wt.%.

The wicking agent preferably has a volume average particle size (D50) from 1 to 250 µm, more preferably from 20 to 200 µm, still more preferably from 30 to 150 µm, most preferably from 50 to 120 µm.

Furthermore, in addition to compounds (a), (b), (c) and optionally (d), a surfactant can be added to the mixture of step (i). Preferably, sodium lauryl sulfate is used as surfactant. Usually, surfactants can be used in an amount of 0.05 to 2 wt.%, preferably of 0.1 to 1.5 wt.%, based on the total weight of the mixture in step (i).

Generally, it is noted that all comments made above regarding components (a), (b), (c) and (d) of the present invention also apply for the processes of the present invention.

The process for producing the pharmaceutical composition of the present invention comprises the steps of
(i) mixing the above illustrated components (a), (b), (c) and optionally (d),
(ii) melting the mixture, optionally cooling off, and
(iii) granulating the melted mixture.

Generally, in step (i) rivaroxaban (a) can be present in an amount of 1 to 70 wt.%, preferably 4 to 40 wt.%, more preferably 5 to 25 wt.%, and particularly preferred between 6 and 20 wt.%, based on the total weight of the mixture resulting from step (i). Generally, in step (i), matrix former (b) can be present in an amount of 1 to 98 wt.%, preferably 5 to 75 wt.%, more preferably 7 to 60 wt.%, and particularly preferred between 10 and 50 wt.%, based on the total weight of the mixture resulting from step (i).

Generally, in step (i), disintegrant (c) can be present in an amount of 1 to 45 wt.%, preferably 5 to 40 wt.%, more preferably 10 to 35 wt.%, and particularly preferred between 10 and 30 wt.%, based on the total weight of the mixture resulting from step (i).

Generally, in step (i), wicking agent (d) can be present in an amount of 0 to 80 wt.%, preferably 5 to 70 wt.%, more preferably 10 to 65 wt.%, and particularly preferred between 15 and 50 wt.%, based on the total weight of the mixture resulting from step (i).

Mixing (i) can be carried out with conventional mixing devices, e.g. in a free fall mixer like Turbula^{®} T 10B (Bachofen AG, Switzerland). Mixing can be carried out e.g. for 1 minute to 1 hour, preferably for 5 to 30 minutes.

In step (ii) the mixture resulting from step (i) is molten. Preferably, rivaroxaban in crystalline form (especially in crystalline form I) is used and the melting conditions are preferably chosen such that rivaroxaban remains in crystalline, especially in crystalline form I.

The specific melting conditions depend on the compounds (a), (b), (c) and optionally (d). Usually, temperatures from 40°C to 200°C, preferably from 60°C to 180°C are used.

In step (iii) the molten mixture resulting from step (ii) is granulated, either in molten state or after having cooled off.

The granulation can for example be carried out by an extrusion process. Hence, steps (ii) and (iii) preferably can be regarded as melt-extrusion process. Generally, the extrusion process should be capable of making essentially spherical particles. Suitable extruders are, for example, screw-feed extruders (axial or endplate, dome and radial) or gravity extruders (cylinder roll, gear roll or radial). Screw-feed extruders are preferred.

The granulation can also for example be carried out by a - preferably heatable - High-Shear-Mixer (e.g. Diosna^{®} P1/6). In this case, steps (i), (ii) and (iii) can be regarded as one process with different sequences of special parameters. The first sequence is step (i) without heating, second sequence is a mixture of step (i) and (ii) with heating, sequence three includes parts of step (ii) and (iii). Preferred parameters of the sequences are dependent upon the chosen components (a), (b), (c) and optionally (d).

In a preferred embodiment the granulation can be carried out with a melt screw extruder (e.g. Leistritz^{®} micro 18), wherein steps (i) and (ii) are unified in one continuous process. Afterwards, the resulting products can be pelletized or granulated. Generally, a temperature gradient is applied, preferably between 80-190 °C.

In a preferred embodiment the granulation conditions in step (iii) are chosen such that the resulting granulated pharmaceutical composition comprises a volume mean particle size (D₅₀) of 10 to 500 µm, more preferably of 50 to 250 µm, further more preferably of 60 to 200 µm, most preferably of 70 to 160 µm.

The bulk density of the granulated pharmaceutical composition made by the process of the present invention generally ranges from of 0.2 to 0.85 g/ml, preferably of from 0.25 to 0.85 g/ml, more preferably of from 0.3 to 0.75 g/ml.

The granulated pharmaceutical composition resulting from step (iii) of the invention preferably possesses Hausner ratios in the range of 1.02 to 1.6, preferably of 1.08 to 1.4, more preferably between 1.10 to 1.3. The Hausner ratio is the ratio of tapped density to bulk density. Bulk density and tapped density are determined according to USP 24, Test 616 "Bulk Density and Tapped Density".

The resulting granulates can be regarded as a "primary pharmaceutical composition", which is suitable for being further processed to an oral dosage form (which represents a "final pharmaceutical composition").

Hence, a further subject of the present invention is an oral dosage form, preferably in form of tablets or in form of a capsule or sachet or stick-pack, containing the above illustrated pharmaceutical composition of the present invention.

An oral dosage form of the present invention generally comprises the (primary) pharmaceutical dosage form according to the present invention and, optionally, pharmaceutical acceptable excipients.

Preferably, the oral dosage form is provided in form of tablets, more preferably film-coated tablets. The tablets preferably are prepared by direct-compression.

Therefore, a further subject of the present invention is a process for producing tablets, comprising the steps of
(i) mixing
   a) rivaroxaban,
   b) a matrix former,
      c1) a disintegrant, and
      d1) optionally a wicking agent,
(ii) melting the mixture,
(iii) granulating the melted mixture,
(iv) mixing the granulate resulting from step (iii) with
   c2) disintegrant,
   d2) optionally a wicking agent, and
   e) optionally further excipients, and
(v) compressing the mixture resulting from step (iv) into tablets.

Process steps (i) to (iii) and components (a) and (b) already have been illustrated above.

Furthermore, all explanations given above for the disintegrant (c) also apply for the first disintegrant portion (c1) as well as the second disintegrant portion (c2). Components (c1) and (c2) can be the same or different disintegrants. Similar, all explanations given above for the wicking agent (d) also apply for the first wicking agent portion (d1) as well as the second wicking agent portion (d2). Components (d1) and (d2) can be the same or different wicking agents.

In step (iv) the granulates resulting from step (iii) (and comprising a first portion of disintegrant c1) are mixed in step (iv) with a second portion of disintegrant (c2) and, optionally, with a second portion of the wicking agent (d2) and, optionally, further excipients. Mixing (iv) can be carried out with conventional mixing devices, e.g. in a free fall mixer like Turbula^{®} T 10B (Bachofen AG, Switzerland). Mixing can be carried out e.g. for 1 minute to 1 hour, preferably for 5 to 30 minutes.

As already mentioned above, the process for producing tablets according to the present invention is characterized by splitting the amount of disintegrant (c) into two portions (c1) and (c2). In a preferred embodiment the weight ratio of component (c1) : component (c2) is from 15 : 85 to 70 : 30, more preferably from 25 : 75 to 60 : 40. In addition, the process for producing tablets according to the present invention preferably can be characterized by splitting the amount of the wicking agent (d) into two portions (d1) and (d2). In a preferred embodiment the weight ratio of component (d1): component (d2) is from 15 : 85 to 70 : 30, more preferably from 25 : 75 to 60 : 40.

Furthermore, if a wicking agent is used, the weight ratio of components (c1) + (c2) : components (d1) + (d2) is preferably from 20 : 80 to 60 : 40, more preferably from 30 : 70 to 50 : 50.

In addition, in the mixing step (iv) preferably one or more further excipient(s), such as fillers, lubricants, glidants and anti-sticking agents can be used. Regarding the above mentioned pharmaceutically acceptable excipients, the application generally refers to "Lexikon der Hilfsstoffe fur Pharmazie, Kosmetik und angrenzende Gebiete", edited by H. P. Fiedler, 5^{th} Edition, Editio Cantor Verlag, Aulendorf and earlier editions, and "Hand-book of Pharmaceutical Excipients", third edition, edited by Arthur H. Kibbe, American Pharmaceutical Association, Washington, USA, and Pharmaceutical Press, London.

Generally, fillers can be used as excipients. Preferred examples of the fillers are soluble and insoluble excipients, like lactose or calcium hydrogen phosphate. The filler is for example present in an amount of 0 to 50 wt.%, preferably of 1 to 20 wt.%, based on the total weight of the tablet core (i.e. in case of film-coated tablets based on the tablet weight without film).

Generally, lubricants can be used as excipients. The lubricant preferably is a stearate or fatty acid, more preferably an earth alkali metal stearate, such as magnesium stearate. The lubricant is suitably present in an amount of 0 to 2 wt.%, preferably about 0.5 to 1.5 wt.%, of the total weight of the tablet core.

Generally, glidants can be used as excipients. The glidant can for example be colloidal silicone dioxide (e.g. Aerosil^{®}). Preferably, the glidant agent is present in an amount of 0 to 8 wt.%, more preferably at 0.1 to 3 wt.% of the total weight of the tablet core.

Generally, anti-sticking agents can be used as excipients. The anti-sticking agent is, for example, talcum and may be present in amounts of 0 to 5 wt.%, more preferably in an amount of 0.5 to 3 wt.%, of the total weight of the tablet core.

In this regard it is generally noted that, due to the nature of pharmaceutical excipients, it cannot be excluded that a certain compound meets the requirements of more than one of the components (b), (c) and (d) or of the above mentioned additional excipients. However, in order to enable an unambiguous distinction, it is preferred in the present application that one and the same pharmaceutical compound can only function as one of the compounds (b) or (c) or (d) or additional excipient. For example, if microcrystalline cellulose functions as wicking agent (d) it cannot additionally function as disintegrant (c) or as filler. Furthermore, in the present application rivaroxaban only functions as component (a) but not as one of components (b), (c) or (d).

The compression step (v), preferably a direct compression step, is preferably carried out on a rotary press, e.g. on a Fette 102i (Fette GmbH, Germany) or a Riva^{®} piccola (Riva, Argentina).

If a rotary press is applied, the main compaction force usually ranges from 1 to 50 kN, preferably from 2 to 40 kN, more preferably from 3.5 to 30 kN.

Finally, subjects of the present inventions are tablets obtainable by any of the processes as described above.

The tablets of the present invention tablets can be film-coated tablets for peroral use or dispersing tablets.

The film-coating agent is for example hydroxypropyl methyl cellulose or methacrylate and may be present in an amount of 1 - 10 wt.%, more preferably in an amount of 2 - 8 wt.%, based on the total weight of the composition.

The pharmaceutical compositions and oral dosage forms (e.g. tablets) of the present invention are formulations showing "immediate release". Within the scope of this patent application, immediate release formulations having a Q value of not less than 75 %, preferably have a Q value of from 80 % to 100 %, more preferably a Q value of from 90 % to 100 %. The Q value is determined as described in USP 32-NF 27 method II (paddle, chapter <711 >). In case of tablets this values refer to the uncoated tablet.

Furthermore, the pharmaceutical compositions and tablets of the present invention preferably do not comprise compounds imparting modified release properties. More preferably, the pharmaceutical compositions and tablets of the present invention do not comprise a modified release system comprising a non-erodible polymer.

In a further aspect, the present invention relates to the use of a combination of crospovidone and a wicking agent for producing an immediate release solid oral dosage form containing rivaroxaban. Preferably, the combination of crospovidone and the wicking agent is a process for producing tablets, more preferably the combination is used intragranularly as well as extragranularly. Also in this aspect the comments given above, e.g. for the amounts and preferred embodiments of the wicking agent, apply.

Finally, the present invention provides the use of the pharmaceutical composition or the oral dosage form of the present invention for the prophylaxis and/or treatment of thromboembolic diseases, such as infarct, angina pectoris (including instable angina) re-occlusions and restenoses after an angioplasty or an aorta-coronary bypass, stroke, transitory ischaemic events, peripheral arterial occlusion, lung embolism or deep vein thrombosis.

The present invention is illustrated by the following examples.

### EXAMPLES

### Example 1: Micronizing rivaroxaban (a)

Crude rivaroxaban (D50 = 130 µm) was micronized on jet air mill and the resulting particle size was determined.

### Sample A Micronization

| | | | |
|---|---|---|---|
| *Feed Rate* | **2.0** g/30" | *Duration* | **12** min |
| *Venturi Pressure* | **12.0** bar | *Mill pressure* | **12.0** bar |
| *Annotations* | **0,039 kg (net) of micronized powder was obtained.** | | |
| *Results* | Analysis report No. | Record 9 | |
| | D10= **0.66** µm | D50 = **1.87** µm | D90 = **4.67** µm |
| | Notes second micronization of trial 1,2,3 | | |

### Example 2: Micronizing rivaroxaban

Crude rivaroxaban (D50 = 130 µm) was micronized on a jet air mill and the resulting particle size was determined.

### Samole B Micronization

| | | | |
|---|---|---|---|
| *Feed Rate* | **10.0** g/30" | *Duration* | **2** min |
| *Venturi Pressure* | **8.**0 bar | *Mill pressure* | **8.0** bar |
| *Annotations* | **0,061 kg (net) of micronized powder was obtained.** | | |
| *Results* | Analysis report No. | Record 8 | |
| | D10= **0.66** µm | D50= **2.47** µm | D90= **7.63** µm |
| | Notes | | |

### Example 3: Process for Producing Tablets

| **Composition** | [mg/DF] |
|---|---|
| Rivaroxaban Polymorph I according to Example 1 | 10 |
| Povidon^{®} VA 64 | 30 |
| Sodium lauryl sulfate | 1.0 |
| Silificied microcrystalline cellulose | 60 |
| Crospovidone | 2 x 10 = 20 |
| Silicium dioxide | 0.4 |
| Magnesium stearate | 0.9 |

Rivaroxaban, Povidon^{®} VA64, sodium lauryl sulfate, 10 mg crospovidone and 10 mg silificied microcrystalline cellulose were premixed in a bin. The premix was heated until melting of the Povidon^{®} VA 64 over a glycerol arrangement for maintaining temperature under granulation. The melt granulate was sieved. The remaining excipients, apart from magnesium stearate, were added and blended for 25 min in a free fall mixer Turbula^{®} TB10. Magnesium stearate was added and blended for further 3 min. The final blend was compressed on a rotary press Riva Piccola.

### Example 4

Dissolution profile and stability data of tablets according to Example 3 have been determined. The determination of the dissolution data has been carried out according to USP (paddle, 900 ml acetate buffer, pH 4.5 + 0.5 % sodium lauryl sulfate, 75 rpm). The dissolution profile of tablets according to the present invention have been compared with tablets of the prior art prepared by wet granulation as disclosed in WO 2005/060940, Example 5.

Furthermore, stability data are determined at 40°C and 75 % relative humidity. All dosage forms were packed in HD-polyethylene.

| **Dissolution [%]** | **initial** | **4 weeks** | **12 weeks** | WO 2005/060940 **initial** |
|---|---|---|---|---|
| 5 min | 75.5 | 76.9 | 75.3 | --- |
| 30 min | 97.7 | 98.0 | 96.6 | 95 |
| 45 min | 99.7 | 100.0 | 98.5 | 96 |
| 60 min | 100.7 | 101.1 | 99.6 | 96 |
| Impurity | | | | |
| Total [%] | 0.14 | 0.14 | 0.14 | |

## Claims

1. Process for producing a pharmaceutical composition, comprising the steps of
(i) mixing
a) rivaroxaban,
b) a matrix former, and
c) a disintegrant,
(ii) melting the mixture, and
(iii) granulating the melted mixture.

2. Process according to claim 1, wherein in step (i) further
d) a wicking agent
is mixed.

3. Process according to claim 1 or 2, wherein the matrix former is a hydrophilic polymer having a weight average molecular weight from 10,000 to 100,000 g/mol.

4. Process according to any one of claims 1 to 3, wherein the disintegrant is selected from sodium carboxymethyl starch, cross-linked polyvinylpyrrolidone, sodium carboxymethyl glycolate and sodium bicarbonate.

5. Process according to any one of claims 1 to 4, wherein in step (i) rivaroxaban, having a volume average particle size (D50) from 0.1 to 5 µm, is used.

6. Process according to any one of claims 2 to 5, wherein the wicking agent is a material with the ability to draw a biological fluid into granulates resulting from step (iii), preferably by physisorption.

7. Process according to any one of claims 1 to 6, wherein in step (i)
a) 5 to 25 wt.% rivaroxaban,
b) 5 to 75 wt.% matrix former,
c) 5 to 35 wt.% disintegrant, and
d) 0 to 80 wt.%, preferably 30 to 60 wt.%, wicking agent
are mixed, based on the total weight of the components used in step (i).

8. Pharmaceutical composition, obtainable by a process as described in any one of claims 1 to 7.

9. Pharmaceutical composition according to claim 8 in form of granulates, having a volume average particle size (D50) from 50 to 250 µm.

10. Oral dosage forms, preferably tablets or capsules, containing a pharmaceutical composition according to claims 8 or 9.

11. Process for producing tablets, comprising the steps of
(i) mixing
a) rivaroxaban,
b) a matrix former,
c1) a disintegrant, and
d1) optionally a wicking agent,
(ii) melting the mixture,
(iii) granulating the melted mixture,
(iv) mixing the granulate resulting from step (iii) with
c2) disintegrant,
d2) optionally wicking agent, and
e) optionally further excipients, and
(v) compressing the mixture resulting from step (iv) into tablets.

12. Process according to claim 11, wherein the weight ratio of component (c1) : component (c2) is from 15 : 85 to 70 : 30.

13. Process according to claim 11 or 12, wherein the weight ratio of components (c1) + (c2) : components (d1) + (d2) is from 20 : 80 to 60 : 40.

14. Tablets obtainable by a process as described in any one of claims 11 to 13.

15. Use of a combination of crospovidone and a wicking agent for producing an immediate release solid oral dosage form containing rivaroxaban.
